(19) Europäisches Patentamt
European Patent Office
Office européen des brevets

(11) **EP 3 804 722 A1**

(12) **EUROPEAN PATENT APPLICATION**
published in accordance with Art. 153(4) EPC


(43) Date of publication:
**14.04.2021 Bulletin 2021/15**

(21) Application number: **19814350.5**

(22) Date of filing: **06.06.2019**

(51) Int Cl.:
***A61K 31/4725*** *(2006.01)* ***A61K 31/416*** *(2006.01)*
***A61K 31/4418*** *(2006.01)* ***A61K 31/4439*** *(2006.01)*
***A61K 31/4704*** *(2006.01)* ***A61P 35/00*** *(2006.01)*

(86) International application number:
**PCT/JP2019/022538**

(87) International publication number:
**WO 2019/235570 (12.12.2019 Gazette 2019/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.06.2018 JP 2018108728**



(71) Applicant: **National University Corporation Hokkaido University**
**Sapporo-shi, Hokkaido 060-0808 (JP)**

(72) Inventors:
- **ISHII Yukiko**
  **Ashigarakami-gun, Kanagawa 258-8577 (JP)**
- **KONDO Toru**
  **Sapporo-shi, Hokkaido 060-0808 (JP)**

(74) Representative: **Godemeyer Blum Lenze Patentanwälte**
**Partnerschaft mbB - werkpatent**
**An den Gärten 7**
**51491 Overath (DE)**


(54) **TREATMENT AGENT AND PHARMACEUTICAL COMPOSITION FOR GLIOMA**


(57) An object of the present invention is to provide an effective treatment agent and a pharmaceutical composition for prevention or treatment of glioma. The present invention provides a glioma treatment agent which comprises a compound represented by formula (1) or a salt thereof.


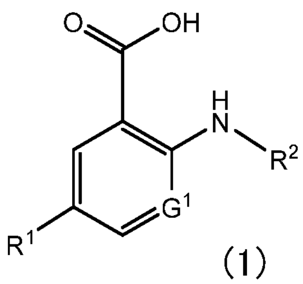

(1)

[Fig. 2]

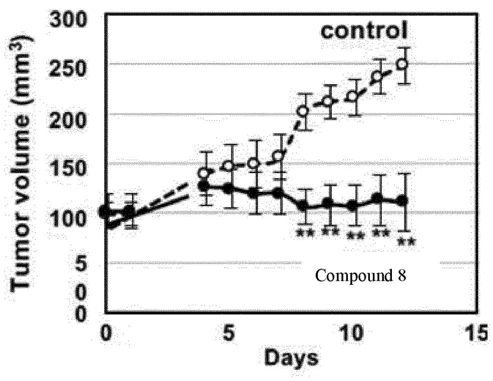


EP 3 804 722 A1

## Description

### Technical Field

The present invention relates to a treatment agent and a pharmaceutical composition for prevention or treatment of glioma, etc.

### Background Art

Glioma is a kind of malignant tumor and is one of the most frequent types of nervous system tumors. Signs and symptoms of glioma are determined by several factors (size, rate of proliferation, tumor localization) and are primarily represented by headache, seizure, neuropathy, and changes in mental status.

The standard treatment for glioma is to remove the tumor as much as possible by surgery and then perform radiation therapy and chemotherapy with temozolomide (TMZ, a glioma standard treatment) which is a DNA alkylating agent. However, since glioma is highly resistant to radiation and drugs such as TMZ, even the standard treatment has hardly improved prognostic survival. One of the causes of this intractableness is thought to be the presence of glioma-initiating cells (GICs) that have replication competence, tumorigenicity, and chemoradiotherapy resistance. The creation of breakthrough treatment methods targeting GIC is awaited. The present inventors established a TMZ-resistant GIC strain (GICR) from a plurality of human GIC strains having tumorigenicity, and identified a compound skeleton with strong cytotoxic activity against GIC/GICR by screening using a low-molecular-weight compound library.

Meanwhile, Patent Document 1 discloses a compound which has an excellent antiproliferative effect on keratinocytes and is useful for preventive or therapeutic treatments of diseases associated with hyperproliferation of keratinocytes, but no studies have been made on the prevention or therapy of glioma.

### Prior Art Documents

### Patent Documents

Patent Document 1: WO 2014/069510

### Summary of Invention

### Object to be Solved by the Invention

As described above, various therapeutic methods for glioma are known, but the effects thereof are not satisfactory. More effective treatment agents and pharmaceutical compositions for prevention or treatment of glioma are desired.

### Means for Solving the Object

Under such circumstances, as a result of intensive studies, the present inventors found that the compound represented by the formula (1) or a salt thereof can solve the above-mentioned problems. These findings have led to the completion of the present invention.

According to the present invention, the following are provided.

[1] A glioma treatment agent which comprises a compound represented by formula (1) or a salt thereof:

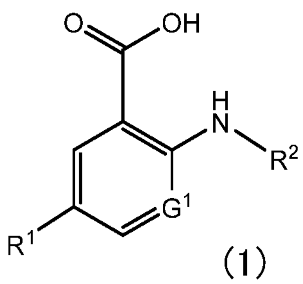

(1)

(wherein

$G^1$ is CH or a nitrogen atom;

$R^1$ is a halogen atom, an optionally substituted $C_{1-6}$ alkyl group, or an optionally substituted $C_{3-8}$ cycloalkyl group;

$R^2$ is an optionally substituted bicyclic condensed hydrocarbon ring group or an optionally substituted bicyclic heterocyclic group, provided that (1) when $R^2$ is an optionally substituted bicyclic condensed hydrocarbon ring group, $G^1$ is a nitrogen atom;

(2) when $G^1$ is CH, and $R^1$ is a chlorine atom or an optionally substituted $C_{3-8}$ cycloalkyl group, $R^2$ is a group represented by formula (2-1) or (2-2)

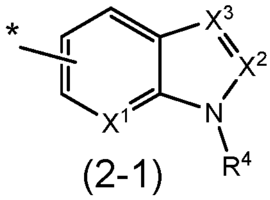
(2-1)

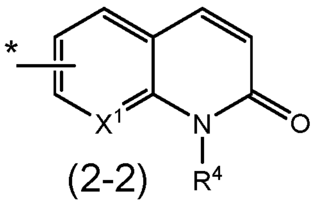
(2-2)

(wherein

$X^1$, $X^2$, and $X^3$ are the same or different and are each $CR^3$ or a nitrogen atom;

$R^3$ is a hydrogen atom, a halogen atom, or an optionally substituted $C_{1-6}$ alkyl group, and $R^4$ is an optionally substituted $C_{3-6}$ alkyl group, an optionally substituted $C_{3-8}$ cycloalkyl group, an optionally substituted $C_{3-8}$ cycloalkyl $C_{1-6}$ alkyl group, an optionally substituted aryl group, an optionally substituted ar-$C_{1-6}$ alkyl group, an optionally substituted acyl group, an optionally substituted heterocyclic group, or an optionally substituted heterocyclic $C_{1-6}$ alkyl group)).

[2] The glioma treatment agent comprising the compound or a salt thereof according to the above [1], wherein $R^1$ is a chlorine atom or an optionally substituted $_{3-8}$ cycloalkyl group.

[3] The glioma treatment agent comprising the compound or a salt thereof according to the above [1] or [2], wherein $R^2$ is a group represented by formula (3-1) or (3-2)

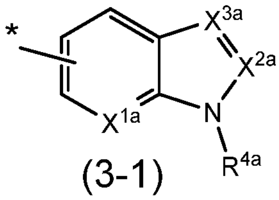
(3-1)

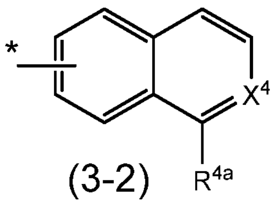
(3-2)

(wherein

$X^{1a}$, $X^{2a}$, and $X^{3a}$ are the same or different and are each $CR^5$ or a nitrogen atom;

$X^4$ is CH or a nitrogen atom;

$R^{4a}$ is an optionally substituted $C_{1-6}$ alkyl group or an optionally substituted aryl group;

$R^5$ is a hydrogen atom, an optionally substituted $C_{3-8}$ cycloalkenyl group, or an optionally substituted aryl group), or

when $G^1$ is CH, and $R^1$ is a chlorine atom or an optionally substituted $C_{3-8}$ cycloalkyl group, $R^2$ is a group represented by formula (4)

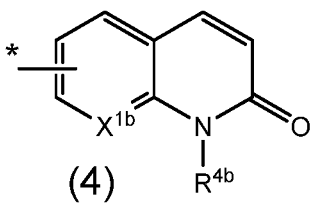
(4)

(wherein

$X^{1b}$ is CH or a nitrogen atom;
$R^{4b}$ is an optionally substituted aryl group).

[4] The glioma treatment agent comprising the compound or a salt thereof according to any one of the above [1] to [3], wherein $G^1$ is a nitrogen atom, $R^2$ is a group represented by formula (5)

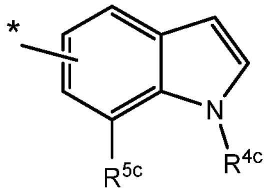

(5)

(wherein

$R^{4c}$ is an optionally substituted $C_{1-6}$ alkyl group;
$R^{5c}$ is an optionally substituted aryl group or an optionally substituted $C_{3-8}$ cycloalkenyl group).

[5] A pharmaceutical composition containing the treatment agent according to any one of the above [1] to [4].
[6] A method for treating glioma, comprising a step of administering the treatment agent according to any one of the above [1] to [4] to a subject.
[7] The treatment agent according to any one of the above [1] to [4], which is used for treating glioma.
[8] A compound represented by formula (1) or a salt thereof, for use in treatment of glioma.
[9] Use of the compound represented by formula (1) or a salt thereof for producing a glioma treatment agent.

Advantageous Effects of Invention

The present invention is useful for treatment such as prevention or therapy of glioma.

Brief Description of Drawings

[Fig. 1] Fig. 1 shows the quantitative results of immunostaining of differentiated and undifferentiated markers in the presence of the compound.
[Fig. 2] Fig 2 shows the tumor-suppressive effect of the compound.
[Fig. 3] Fig. 3 shows the cancellation of the decrease in SOX2 expression by the addition of UDP-GlcNAc. GlcNAc represents N-acetyl-D-glucosamine.
[Fig. 4] Fig. 4 shows the neutralization of GIC-damaging activity by the addition of UDP-GlcNAc.
[Fig. 5] Fig. 5 shows the neutralization of GIC-damaging activity by the SOX2 mutant.
[Fig. 6] Fig. 6 shows the inhibition of GIC growth and SOX2 expression by OSMI-1.
[Fig. 7] Fig. 7 shows changes in metabolites in GICs due to the addition of the compound.

Embodiment of Carrying out the Invention

Hereinafter, the present invention will be described in detail.
In the present invention, unless otherwise specified, each term has the following meaning.
The term "halogen atom" used herein refers to a fluorine atom, a chlorine atom, a bromine atom, or an iodine atom.
The term "$C_{3-6}$ alkyl group" used herein refers to linear or branched $C_{3-6}$ alkyl groups such as propyl, isopropyl, butyl, sec-butyl, isobutyl, tert-butyl, pentyl, isopentyl, and hexyl groups.
The term "$C_{1-6}$ alkyl group" used herein refers to linear or branched $C_{1-6}$ alkyl groups such as methyl, ethyl, propyl, isopropyl, butyl, sec-butyl, isobutyl, tert-butyl, pentyl, isopentyl, and hexyl groups.
The term "$C_{2-6}$ alkenyl group" used herein refers to linear or branched $C_{2-6}$ alkenyl groups such as vinyl, allyl, propenyl, isopropenyl, butenyl, isobutenyl, 1,3-butadienyl, pentenyl, and hexenyl groups.

The term "$C_{3-8}$ cycloalkyl group" used herein refers to $C_{3-8}$ cycloalkyl groups such as cyclopropyl, cyclobutyl, cyclopentyl, and cyclohexyl groups.

The term "$C_{3-8}$ cycloalkenyl group" used herein refers to $C_{3-8}$ cycloalkenyl groups such as cyclopropenyl, cyclobutenyl, cyclopentenyl, cyclohexenyl, and cyclohexanedienyl groups.

The term "$C_{3-8}$ cycloalkyl $C_{1-6}$ alkyl group" used herein refers to $C_{3-8}$ cycloalkyl $C_{1-6}$ alkyl groups such as cyclopropylmethyl, 2-(cyclopropyl)ethyl, cyclobutylmethyl, 2-(cyclobutyl)ethyl, cyclopentylmethyl, and cyclohexylmethyl groups.

The term "aryl group" used herein refers to a phenyl group, a bicyclic condensed hydrocarbon ring group, or a tricyclic condensed hydrocarbon ring group.

The term "ar-$C_{1-6}$ alkyl group" used herein refers to a methoxy, ethoxy, propoxy, or isopropoxy group.

The term "acyl group" used herein refers to a formyl group, succinyl group, glutaryl group, male oil group, phthaloyl group, $C_{2-12}$ alkanoyl group, aroyl group, heterocyclic carbonyl group, or ($\alpha$-substituted) aminoacetyl group.

The term "$C_{2-12}$ alkanoyl group" used herein refers to linear or branched $C_{2-12}$ alkanoyl groups such as acetyl, propionyl, valeryl, isovaleryl, and pivaloyl groups.

The term "aloyl group" used herein refers to a benzoyl or naphthoyl group.

The term "heterocyclic carbonyl group" used herein refers to a nicotinoyl, thenoyl, pyrrolidinocarbonyl, or furoyl group.

The term "($\alpha$-substituted) aminoacetyl group" used herein refers to ($\alpha$-substituted) aminoacetyl groups in which the N-terminus derived from amino acids (including amino acids such as glycine, alanine, valine, leucine, isoleucine, serine, Threonine, cysteine, methionine, aspartic acid, glutamic acid, asparagine, glutamine, arginine, lysine, histidine, hydroxylysine, phenylalanine, tyrosine, tryptophan, proline, and hydroxyproline) may be protected.

The term "bicyclic condensed hydrocarbon ring group" used herein refers to a bicyclic condensed hydrocarbon ring in which a part such as a pentalenyl, indanyl, indenyl, or naphthyl group may be hydrogenated.

The term "tricyclic condensed hydrocarbon ring group" used herein refers to a tricyclic condensed hydrocarbon ring in which a part such as a biphenylenyl, acenaphthenyl, acenaphthylenyl, fluorenyl, phenalenyl, phenanthrenyl, or anthracenyl group may be hydrogenated.

The term "heterocyclic group" used herein refers to a monocyclic heterocyclic group, a bicyclic heterocyclic group, or a tricyclic heterocyclic group.

The term "monocyclic heterocyclic group" used herein refers to a monocyclic nitrogen-containing heterocyclic group, a monocyclic oxygen-containing heterocyclic group, a monocyclic sulfur-containing heterocyclic group, a monocyclic nitrogen/oxygen-containing heterocyclic group, or a monocyclic nitrogen/sulfur - containing heterocyclic group.

The term "monocyclic oxygen-containing heterocyclic group" used herein refers to a tetrahydrofuranyl, furanyl, tetrahydropyranyl, dihydropyranyl, or pyranyl group.

The term "monocyclic sulfur-containing heterocyclic group" used herein refers to a thienyl group.

The term "monocyclic nitrogen/oxygen-containing heterocyclic group" used herein refers to monocyclic nitrogen/oxygen-containing heterocyclic groups containing only a nitrogen atom and an oxygen atom as heteroatoms forming the ring, such as oxazolyl, isooxazolyl, oxadiazolyl, and morpholinyl groups.

The term "monocyclic nitrogen/sulfur-containing heterocyclic group" used herein refers to monocyclic nitrogen/sulfur-containing heterocyclic groups containing only a nitrogen atom and a sulfur atom as heteroatoms forming the ring, such as thiazolyl, isothiazolyl, thiadiazolyl, thiomorpholinyl, 1-oxide thiomorpholinyl and 1,1-dioxide thiomorpholinyl groups.

The term "bicyclic heterocyclic group" used herein refers to a bicyclic nitrogen-containing heterocyclic group, a bicyclic oxygen-containing heterocyclic group, a bicyclic sulfur-containing heterocyclic group, a bicyclic nitrogen/oxygen-containing heterocyclic group, or a bicyclic nitrogen/sulfur-containing heterocyclic group.

The term "bicyclic nitrogen-containing heterocyclic group" used herein refers to bicyclic nitrogen-containing heterocyclic groups containing only an nitrogen atom as a heteroatom forming the ring, such as indolinyl, indolyl, isoindolinyl, isoindolyl, pyrrolopyridinyl, indazolyl, benzoimidazolyl, benzotriazolyl, tetrahydroquinolinyl, dihydroquinolinyl, quinolinyl, tetrahydroquinolinyl, tetrahydroisoquinolinyl, isoquinolinyl, dihydroquinazolinyl, cinnolinyl, phthalazinyl, quinazolinyl, dihydroquinoxalinyl, quinoxalinyl, naphthyridinyl, purinyl, pteridinyl, and quinuclidinyl groups.

The term "bicyclic oxygen-containing heterocyclic group" used herein refers to bicyclic oxygen-containing heterocyclic groups containing only an oxygen atom as a heteroatom forming the ring, such as 2,3-dihydrobenzofuranyl, benzofuranyl, isobenzofuranyl, chromanyl, chromenyl, isochromanyl, 1,3-benzodioxolyl, 1,3-benzodioxanyl, and 1,4-benzodioxanyl groups.

The term "bicyclic sulfur-containing heterocyclic group" used herein refers to bicyclic sulfur-containing heterocyclic groups containing only a sulfur atom as a heteroatom forming the ring, such as 2,3-dihydrobenzothienyl and benzothienyl groups.

The term "bicyclic nitrogen/oxygen-containing heterocyclic group" used herein refers to bicyclic nitrogen/oxygen-containing heterocyclic groups containing only a nitrogen atom and an oxygen atom as heteroatoms forming the ring,

such as dihydrobenzoxazolyl, benzoxazolyl, benzisooxazolyl, benzoxadiazolyl, benzomorpholinyl, dihydropyranopyridyl, dihydrodioxinopyridyl, and dihydropyridoxazinyl groups.

The term "bicyclic nitrogen/sulfur-containing heterocyclic group" used herein refers to bicyclic nitrogen/sulfur-containing heterocyclic groups containing a nitrogen atom and a sulfur atom as heteroatoms forming the ring, such as dihydrobenzothiazolyl, benzothiazolyl, benzisothiazolyl, and benzothiadiazolyl groups.

The term "heterocyclic $C_{1-6}$ alkyl group" used herein refers to: monocyclic nitrogen-containing heterocyclic $C_{1-6}$ alkyl groups such as azetidinylmethyl, azetidinylethyl, pyrrolidinylmethyl, pyrrolidinylethyl, piperidylmethyl, piperidylethyl, pyridylmethyl, pyridylethyl, imidazolylmethyl, imidazolylethyl, piperazinylmethyl, and piperazinylethyl groups; monocyclic oxygen-containing heterocyclic $C_{1-6}$ alkyl groups such as tetrahydrofuranylmethyl and tetrahydropyranylmethyl; monocyclic sulfur-containing heterocyclic $C_{1-6}$ alkyl groups such as a thienylmethyl group; monocyclic nitrogen/oxygen-containing heterocyclic $C_{1-6}$ alkyl groups such as oxazolylmethyl, oxazolylethyl, isoxazolylmethyl, isoxazolylethyl, morphorinylmethyl, and morphorinylethyl groups; monocyclic nitrogen/sulfur-containing heterocyclic $C_{1-6}$ alkyl groups such as thiazolyl methyl, thiazolyl ethyl, isothiazolyl methyl, and isothiazolyl ethyl groups; bicyclic nitrogen-containing heterocyclic $C_{1-6}$ alkyl groups such as indolylmethyl, indolylethyl, benzimidazolylmethyl, benzimidazolylethyl, quinolylmethyl, and quinolylethyl groups; bicyclic oxygen-containing heterocyclic $C_{1-6}$ alkyl groups such as benzofuranylmethyl, isobenzofuranylmethyl, and cromanylmethyl groups; bicyclic sulfur-containing heterocyclic $C_{1-6}$ alkyl groups such as a benzothienylmethyl group; bicyclic nitrogen/oxygen-containing heterocyclic $C_{1-6}$ alkyl groups such as benzoxazolylmethyl and benzoisoxazolylmethyl groups; bicyclic nitrogen/sulfur-containing heterocyclic $C_{1-6}$ alkyl groups such as benzothiazolylmethyl and benzoisothiazolylmethyl groups; tricyclic nitrogen-containing heterocyclic $C_{1-6}$ alkyl groups such as a carbazolylmethyl group; tricyclic oxygen-containing heterocyclic $C_{1-6}$ alkyl groups such as a xanthenylmethyl group; and tricyclic sulfur-containing heterocyclic $C_{1-6}$ alkyl groups such as a thianthrenyl methyl group.

Examples of a salt of the compound of formula (1) may include salts with commonly known basic groups such as amino groups or acidic groups such as phenolic hydroxyl groups or carboxyl groups.

Examples of salts of basic groups may include: salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of salts of acidic groups may include: salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N, N-dimethylaniline, N-methylpiperidine, N-methylmorpholine, diethylamine, dicyclohexylamine, procaine, dibenzylamine, N-benzyl-p-phenethylamine, 1-ephenamine, and N, N'-dibenzylethylenediamine.

Glioma is a malignant tumor that develops in the brain and is classified into four grades (grades I to IV) according to the standards of the World Health Organization (WHO). Grade III and IV gliomas are referred to as "high-grade glioma" and have high proliferative and infiltrative potential. Among high-grade gliomas, the most malignant one is Grade IV glioblastoma. The term "glioma" used herein shall include all grades of gliomas and high-grade gliomas and glioblastomas.

The "treatment" of the present invention includes prevention or therapy. Prevention includes the inhibition of onset, reduction of risk of onset, and delay of onset. Therapy includes the improvement (maintenance or delay) of the disease or condition of interest or suppression of progression of the same. Subjects of treatment include humans or non-human animals in need of the treatment.

The "agent" of the present invention can be a composition in which a compound or a salt thereof, which is an active ingredient, is appropriately mixed with formulation additives such as an excipient, a carrier, and a diluent used for formulation. The "agent" may contain other active ingredients and may be used with a medicine containing other active ingredients.

Among the compounds of the present invention, the following compounds are preferable.

$G^1$ is CH or a nitrogen atom.

$R^1$ is a halogen atom, an optionally substituted $C_{1-6}$ alkyl group, or an optionally substituted $C_{3-8}$ cycloalkyl group, and is preferably a chlorine atom or an optionally substituted $C_{3-8}$ cycloalkyl group, more preferably a chlorine atom or an optionally substituted cyclopropyl group, and further preferably a chlorine atom or a cyclopropyl group.

Examples of a substituent in a $C_{1-6}$ alkyl group or a $C_{3-8}$ cycloalkyl group for $R^1$ may include at least one group selected from the substituent group $\alpha$.

$R^2$ is an optionally substituted bicyclic condensed hydrocarbon ring group or an optionally substituted bicyclic heterocyclic group. Examples of a substituent in a bicyclic condensed hydrocarbon ring group or a bicyclic heterocyclic group for $R^2$ may include at least one group selected from the substituent group $\alpha$.

Provided that when R2 is an optionally substituted bicyclic condensed hydrocarbon ring group, $G^1$ is a nitrogen atom,

and when $G^1$ is CH, and $R^1$ is a chlorine atom or an optionally substituted $C_{3-8}$ cycloalkyl group, $R^2$ is a group represented

by the following formula (2-1) or (2-2).

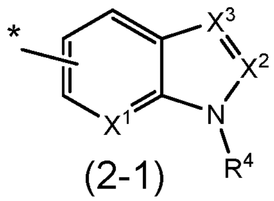

(2-1)

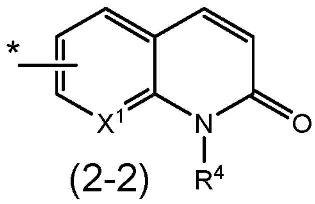

(2-2)

In the formula, $X^1$, $X^2$, and $X^3$ are the same or different and are each $CR^3$ or a nitrogen atom; $R^3$ is a hydrogen atom, a halogen atom, or an optionally substituted $C_{1-6}$ alkyl group, and $R^4$ is an optionally substituted $C_{3-6}$ alkyl group, an optionally substituted $C_{3-8}$ cycloalkyl group, an optionally substituted $C_{3-8}$ cycloalkyl $C_{1-6}$ alkyl group, an optionally substituted aryl group, an optionally substituted ar-$C_{1-6}$ alkyl group, an optionally substituted acyl group, an optionally substituted heterocyclic group, or an optionally substituted heterocyclic $C_{1-6}$ alkyl group.

Examples of a substituent in a $C_{1-6}$ alkyl group for $R^3$ may include at least one group selected from the substituent group $\alpha$.

Examples of a substituent in a $C_{3-6}$ alkyl group, a $C_{3-8}$ cycloalkyl group, a $C_{3-8}$ cycloalkyl $C_{1-6}$ alkyl group, an aryl group, ar-$C_{1-6}$ alkyl group, an acyl group, a heterocyclic group, or a heterocyclic $C_{1-6}$ alkyl group for $R^4$ may include at least one group selected from the substituent group $\alpha$.

$R^2$ is preferably a group represented by the following formula (3-1) or (3-2).

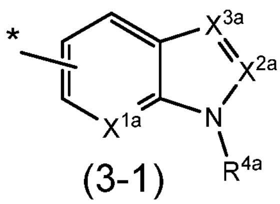

(3-1)

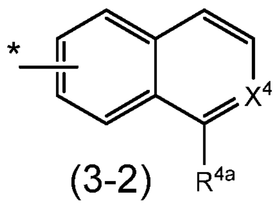

(3-2)

In the formula, $X^{1a}$, $x^{2a}$, and $X^{3a}$ are the same or different and are each $CR^5$ or a nitrogen atom; $X^4$ is CH or a nitrogen atom; $R^{4a}$ is an optionally substituted $C_{1-6}$ alkyl group or an optionally substituted aryl group; $R^5$ is a hydrogen atom, an optionally substituted $C_{3-8}$ cycloalkenyl group, or an optionally substituted aryl group.

Alternatively, when $G^1$ is CH, and $R^1$ is a chlorine atom or an optionally substituted $C_{3-8}$ cycloalkyl group, $R^2$ is preferably a group represented by the following formula (4).

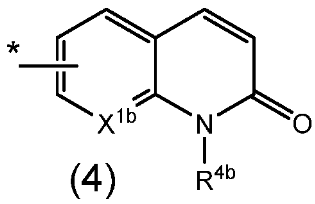

(4)

In the formula, $X^{1b}$ is CH or a nitrogen atom; $R^{4b}$ is an optionally substituted aryl group.

A $C_{1-6}$ alkyl group for $R^{4a}$ is preferably a $C_{1-4}$ alkyl group and more preferably a methyl group.

An aryl group for $R^{4a}$ is preferably a phenyl group.

Examples of a substituent in a $C_{1-6}$ alkyl group or an aryl group for $R^{4a}$ may include at least one group selected from the substituent group $\alpha$.

A $C_{3-8}$ cycloalkenyl group for $R^5$ is preferably a $C_{3-6}$ cycloalkenyl group and more preferably a cyclohexenyl group.

An aryl group for $R^5$ is preferably a phenyl group.

Examples of a substituent in a $C_{3-8}$ cycloalkenyl group, an aryl group, or a heterocyclic group for $R^5$ may include at least one group selected from the substituent group $\alpha$.

An aryl group for $R^{4b}$ is preferably a phenyl group.

Examples of a substituent in an aryl group for $R^{4b}$ may include at least one group selected from the substituent group $\alpha$.

$R^2$ is preferably a group represented by the formula (5). In this case, $G^1$ is preferably a nitrogen atom.

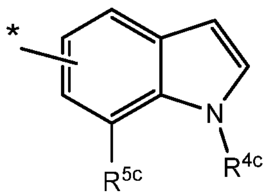

(5)

In the formula, $R^{4c}$ is an optionally substituted $C_{1-6}$ alkyl group; $R^{5c}$ is an optionally substituted aryl group or an optionally substituted $C_{3-8}$ cycloalkenyl group.

A $C_{1-6}$ alkyl group for $R^{4c}$ is preferably a $C_{1-4}$ alkyl group and more preferably a methyl group.

Examples of a substituent in a $C_{1-6}$ alkyl group for $R^{4c}$ may include at least one group selected from the substituent group $\alpha$.

An aryl group for $R^{5c}$ is preferably a phenyl group.

A $C_{3-8}$ cycloalkenyl group for $R^{5c}$ is preferably a $C_{3-6}$ cycloalkenyl group and more preferably a cyclohexenyl group.

Examples of a substituent in an aryl group or a $C_{3-8}$ cycloalkenyl group for $R^{5c}$ may include at least one group selected from the substituent group $\alpha$.

Substituent group $\alpha$: halogen atom, optionally protected hydroxyl group, optionally protected carboxyl group, optionally protected amino group, nitro group, cyano group, carbamoyl group which may be substituted with at least one group selected from the substituent group $\beta$, $C_{1-6}$alkyl group which may be substituted with at least one group selected from the substituent group $\beta$, $C_{2-6}$alkenyl group which may be substituted with at least one group selected from the substituent group $\beta$, $C_{3-8}$cycloalkyl group which may be substituted with at least one group selected from the substituent group $\beta$, $C_{1-6}$alkoxy group which may be substituted with at least one group selected from the substituent group $\beta$, acyl group which may be substituted with at least one group selected from the substituent group $\beta$, alkoxycarbonyl group which may be substituted with at least one group selected from the substituent group $\beta$, $C_{1-6}$alkylamino group which may be substituted with at least one group selected from the substituent group $\beta$, di($C_{1-6}$alkyl)amino group which may be substituted with at least one group selected from the substituent group $\beta$, $C_{1-6}$alkylthio group which may be substituted with at least one group selected from the substituent group $\beta$, $C_{1-6}$alkylsulfonyl group which may be substituted with at least one group selected from the substituent group $\beta$, aryl group, heterocyclic group which may be substituted with at least one group selected from the substituent group $\beta$, and oxo group.

Substituent group $\beta$: halogen atom, optionally protected hydroxy group, optionally protected carboxyl group, optionally protected amino group, carbamoyl group, $C_{1-6}$alkyl group which may be substituted with a halogen atom, $C_{1-6}$alkoxy group which may be substituted with a halogen atom, $C_{1-6}$alkylamino group, di($C_{1-6}$alkyl)amino group, heterocyclic group, and oxo group.

The following compounds can be mentioned as preferable compounds in the present invention: 2-((1-benzyl-1H-indazole-5-yl)amino)-5-cyclopropylbenzoate, 2-((1-(cyclohexylmethyl)-1H-indole-5-yl)amino)-5-cyclopropylnicotinic acid, 5-cyclopropyl-2-((1-phenyl-1H-indole-4-yl)amino)nicotinic acid, 5-cyclopropyl-2-((7-(2-fluorophenyl)-1-methyl-1H-indole-5-yl)amino)nicotinic acid, 5-cyclopropyl-2-((1-methyl-7-(4-trifluoromethyl)phenyl)-1H-indole-5-yl)amino)nicotinic acid, 2-((7-(3-chlorophenyl)-1-methyl-1H-indole-5-yl)amino)-5-cyclopropylnicotinic acid, 5-cyclopropyl-2-((1-methyl-7-(4-(trifluoromethoxy)phenyl)-1H-indole-5-yl)amino)nicotinic acid, 5-cyclopropyl-2-((1-phenyl-1H-indole-5-yl)amino)nicotinic acid, 5-cyclopropyl-2-((5-phenylnaphthalene-1-yl)amino)nicotinic acid, 5-cyclopropyl-2-((5-phenylnaphthalene-2-yl)amino)nicotinic acid, 5-cyclopropyl-2-((1-methyl-7-phenyl-1H-indole-5-yl)amino)nicotinic acid, 5-cyclopropyl-2-((1-(3-fluorophenyl)-1H-indole-5-yl)amino)nicotinic acid, 2-((1-benzyl-6-fluoro-1H-indole-5-yl)amino)-5-cyclopropylnicotinic acid, 2-((1-benzyl-4,6-difluoro-1H-indole-5-yl)amino)-5-cyclopropylnicotinic acid, 5-cyclopropyl-2-((7-(3,6-dihydro-2H-pyran-4-yl)-1-methyl-1H-indole-5-yl)amino)nicotinic acid, 5-cyclopropyl-2-((1-phenylisoquinoline-6-yl)amino)nicotinic acid, 2-((7-(4-cyanophenyl)-1-methyl-1H-indole-5-yl)amino)-5-cyclopropylnicotinic acid, 2-((7-(4-chlorophenyl)-1-methyl-1H-indole-5-yl)amino)-5-cyclopropylnicotinic acid, 5-cyclopropyl-2-((2-oxo-1-phenyl-1,2-dihydroquinoline-5-yl)amino)benzoic acid, 2-((7-cyclohex-1-en-1-yl)-1-methyl-1H-indole-5-yl)amino)-5-cyclopropylnicotinic acid, and 5-cyclopropyl-2-((1-(3-fluorobenzyl)-1H-indole-5-yl)amino)nicotinic acid.

The compound of the present invention is preferably at least one compound selected from 5-cyclopropyl-2-((7-(2-fluorophenyl)-1-methyl-1H-indole-5-yl)amino)nicotinic acid, 5-cyclopropyl-2-((1-methyl-7-(4-trifluoromethyl)phenyl)-1H-indole-5-yl)amino)nicotinic acid, 2-((7-(3-chlorophenyl)-1-methyl-1H-indole-5-yl)amino)-5-cyclopropylnicotinic acid, 5-cyclopropyl-2-((1-methyl-7-(4-(trifluoromethoxy)phenyl)-1H-indole-5-yl)amino)nicotinic acid, 5-cyclopropyl-2-((5-phenylnaphthalene-l-yl)amino)nicotinic acid, 5-cyclopropyl-2-((5-phenylnaphthalene-2-yl)amino)nicotinic acid, 5-cyclopropyl-2-((1-methyl-7-phenyl-1H-indole-5-yl)amino)nicotinic acid, 5-cyclopropyl-2-((1-phenylisoquinoline-6-yl)amino)nicotinic acid, 2-((7-(4-cyanophenyl)-1-methyl-1H-indole-5-yl)amino)-5-cyclopropylnicotinic acid, 2-((7-(4-chlorophenyl)-1-methyl-1H-indole-5-yl)amino)-5-cyclopropylnicotinic acid, 5-cyclopropyl-2-((2-oxo-1-phenyl-1,2-dihydroquinoline-5-yl)amino)benzoic acid, and 2-((7-cyclohex-1-en-1-yl)-1-methyl-1H-indole-5-yl)amino)-5-cyclopropylnicotinic acid, or a salt thereof.

The compound of the present invention is further preferably at least one compound selected from 5-cyclopropyl-2-((1-methyl-7-(4-trifluoromethyl)phenyl)-1H-indole-5-yl)amino)nicotinic acid, 5-cyclopropyl-2-((1-methyl-7-(4-(trifluoromethoxy)phenyl)-1H-indole-5-yl)amino)nicotinic acid, 2-((7-(4-chlorophenyl)-1-methyl-1H-indole-5-yl)amino)-5-cyclopropylnicotinic acid, and 2-((7-cyclohex-1-en-1-yl)-1-methyl-1H-indole-5-yl)amino)-5-cyclopropylnicotinic acid, or a salt thereof.

5-Cyclopropyl-2-((1-phenyl-1H-indole-5-yl)amino)nicotinic acid and 5-cyclopropyl-2-((1-(3-fluorobenzyl)-1H-indole-5-yl)amino)nicotinic acid are also preferable as the compound of the present invention.

Preferred salts of the present invention include pharmacologically acceptable salts.

In a case in which isomers (for example, optical isomers, geometric isomers, and tautomers) are present in the compound represented by the formula (1), the present invention includes those isomers and also includes solvates, hydrates, and crystals of various shapes.

Next, a method for producing the compound of the present invention will be described.

The compound of the present invention can be produced according to the production method described in WO2014/069510, but is not limited thereto.

The term "pharmaceutical composition" used herein refers to a composition in which a compound or a salt thereof, which is an active ingredient, is appropriately mixed with formulation additives such as an excipient, a carrier, and a diluent used for formulation.

In a case in which the present invention is used for a pharmaceutical composition, the pharmaceutical composition may be appropriately mixed with formulation additives such as an excipient, a carrier, and a diluent generally used for formulation.

Examples of additives may include excipients, disintegrants, binders, lubricants, taste masking agents, colorants, flavoring agents, surfactants, coatings, and plasticizers.

Examples of excipients may include: sugar alcohols such as erythritol, mannitol, xylitol, and sorbitol; sugars such as sucrose, powdered sugar, lactose, and glucose; cyclodextrins such as α-cyclodextrin, β-cyclodextrin, and sodium sulfobutyl ether β-cyclodextrin; celluloses such as crystalline cellulose and microcrystalline cellulose; and starches such as corn starch, potato starch and pregelatinized starch.

Examples of disintegrants may include carmellose, carmellose calcium, croscarmellose sodium, sodium carboxymethyl starch, crospovidone, low substituted hydroxypropylcellulose, and partially pregelatinized starch.

Examples of binders may include Hydroxypropyl cellulose, carmellose sodium, and methyl cellulose.

Examples of lubricants may include stearic acid, magnesium stearate, calcium stearate, talc, hydrous silicon dioxide, light anhydrous silicic acid, and sucrose fatty acid ester.

Examples of taste masking agents may include aspartame, saccharin, stevia, thaumatin, and acesulfame potassium.

Examples of colorants may include titanium dioxide, iron sesquioxide, yellow iron sesquioxide, black iron oxide, Food Red No. 102, Food Yellow No. 4, and Food Yellow No. 5.

Examples of flavoring agents may include essential oils such as orange oil, lemon oil, peppermint oil, and pine oil; essences such as orange essence and peppermint essence; flavors such as cherry flavors, vanilla flavors and fruit flavors; powdered fragrances such as apple micron, banana micron, peach micron, strawberry micron and orange micron; vanillin; and ethyl vanillin.

Examples of surfactants may include sodium lauryl sulfate, sodium sulfosuccinate dioctyl, polysorbate, and polyoxyethylene hydrogenated castor oil.

Examples of coatings may include hydroxypropyl methylcellulose, aminoalkyl methacrylate copolymer E, aminoalkyl Methacrylate Copolymer RS, ethyl cellulose, cellulose phthalate acetate, hydroxypropyl methylcellulose phthalate, methacrylic acid copolymer L, methacrylic acid copolymer LD, and methacrylic acid copolymer S.

Examples of plasticizers may include triethyl citrate, macrogol, triacetin, and propylene glycol.

These additives may be used alone or in combination of two or more kinds.

The blending amounts thereof are not particularly limited, and they may be blended appropriately such that their effects are sufficiently exhibited according to their purposes.

They may be administered orally or parenterally in the form of tablets, capsules, powders, syrups, granules, pills, suspensions, emulsions, liquids, powdered preparations, suppositories, eye drops, nasal drops, ear drops, patches, ointments, injections, or the like in accordance with an ordinary method. In addition, the administration method, dose, and frequency of administration can be appropriately selected according to the age, body weight, and symptoms of the patient.

Usually, for adults, 0.01 to 1000 mg/kg may be administered in one to several divided doses daily by oral or

parenteral administration.

Examples

The present invention is explained with reference to the Examples below; however, the present invention is not limited to the Examples.

[Production of Compound Nos. 1 to 20]

The compounds listed in Tables 1 to 3 were produced according to the production method described in WO 2014/069510.

[Table 1]

| Co. No. | Structural Formula | Compound Name |
|---|---|---|
| 1 | 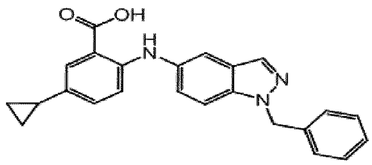 | 2-((1-benzyl-1h-indazole-5-yl)amino)-5-cyclopropylbenzoate |
| 2 | 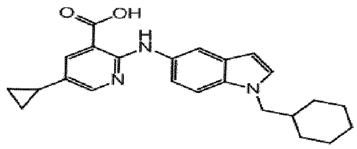 | 2-((1-(cyclohexylmethyl)-1H-indole-5-yl)amino)-5-cyclopropylnicotinic acid |
| 3 | 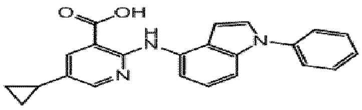 | 5-cyclopropyl-2-((1-phenyl-1H-indole-4-yl)amino)nicotinic acid |
| 4 | 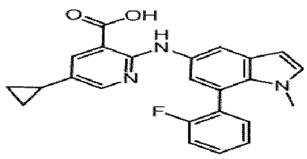 | 5-cyclopropyl-2-((7-(2-fluorophenyl)-1-methyl-1H-indole-5-yl) amino)nicotinic acid |
| 5 | 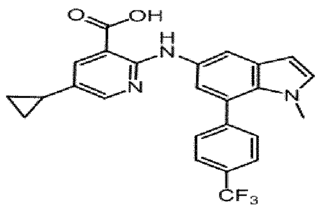 | 5-cyclopropyl-2-((1-methyl-7-(4-tnfluoromethyl)phenyl)-1H-indole-5-yl)amino)nicotinic acid |
| 6 | 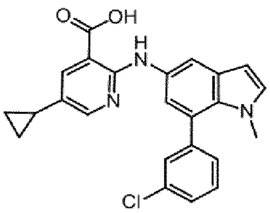 | 2-((7-(3-chlorophenyl)-1-methyl-1H-indole-5-yl)amino)-5-cyclopropylnicotinic acid |

(continued)

| Co. No. | Structural Formula | Compound Name |
|---|---|---|
| 7 | 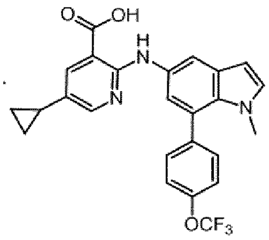 | 5-cyclopropyl-2-((1-methyl-7-(4-(trifluoromethoxy)phenyl)-1H-indole-5-yl)amino)nicotinic acid |

[Table 2]

| Co. No. | Structural Formula | Compound Name |
|---|---|---|
| 8 | 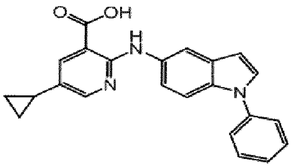 | 5-cyclopropyl-2-((1-phenyl-1H-indole-5-yl)amino)nicotinic acid |
| 9 | 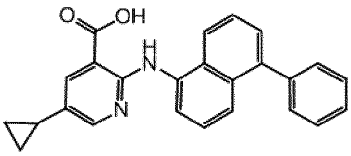 | 5-cyclopropyl-2-((5-phenylnaphthalene-1-yl)amino)nicotinic acid |
| 10 | 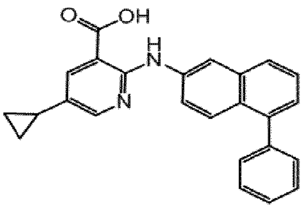 | 5-cyclopropyl-2-((5-phenylnaphthalene-2-yl)amino)nicotinic acid |
| 11 | 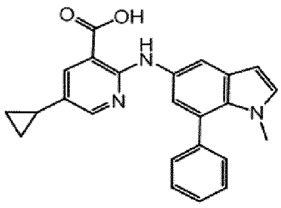 | 5-cyclopropyl-2-((1-methyl-7-phenyl-1H-indole-5-yl)amino)nicotinic acid |
| 12 | 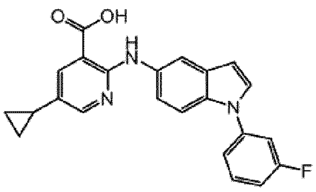 | 5-cyclopropyl-2-((1-(3-fluorophenyl)-1H-indole-5-yl)amino)nicotinic acid |
| 13 | 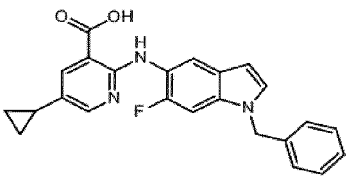 | 2-((1-benzyl-6-fluoro-1H-indole-5-yl)amino)-5-cyclopropylnicotinic acid |

(continued)

| Co. No. | Structural Formula | Compound Name |
|---|---|---|
| 14 | 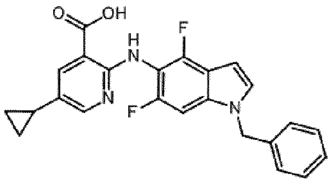 | 2-((1-benzyl-4,6-difluoro-1H-indole-5-yl)amino)-5-cyclopropylnicotinic acid |

[Table 3]

| Co. No. | Structural Formula | Compound Name |
|---|---|---|
| 15 | 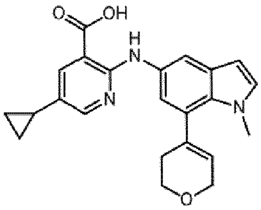 | 5-cyclopropyl-2-((7-(3,6-dihydro-2H-pyran-4-yl)-1-methyl-1H-indole-5-yl)amino)nicotinic acid |
| 16 | 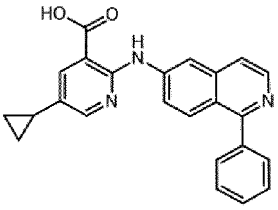 | 5-cyclopropyl-2-((1-phenylisoquinoline-6-yl)amino)nicotinic acid |
| 17 | 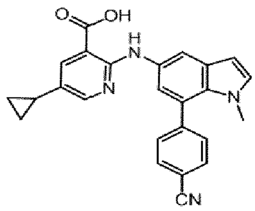 | 2-((7-(4-cyanophenyl)-1-methyl-1H-indole-5-yl)amino)-5-cyclopropylnicotinic acid |
| 18 | 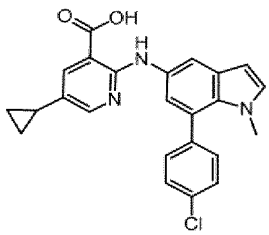 | 2-((7-(4-chlorophenyl)-1-methyl-1H-indole-5-yl)amino)-5-cyclopropylnicotinic acid |
| 19 | 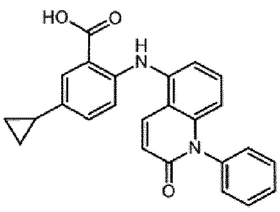 | 5-cyclopropyl-2-((2-oxo-1-phenyl-1,2-dihydroquinoline-5-yl) amino)benzoic acid |

(continued)

| Co. No. | Structural Formula | Compound Name |
|---|---|---|
| 20 | 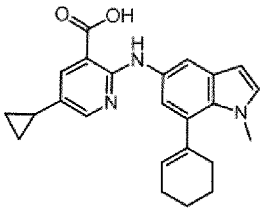 | 2-((7-cyclohex-1-en-1-yl)-1-methyl-1H-indole-5-yl)amino)-5-cyclopropylnicotinic acid |
| 21 | 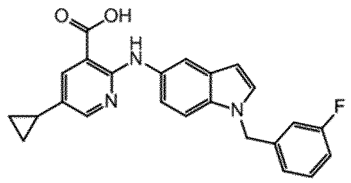 | 5-cyclopropyl-2-((1-(3-fluorobenzyl)-1H-indole-5-yl)amino) nicotinic acid |

[Cell growth test]

Abbreviations used herein are shown below.

DMEM: Dulbecco's modified Eagle's medium
bFGF: Basic fibroblast growth factor
EGF: Epidermal growth factor
FCS: Fetal calf serum
MTT: 3-[4,5-dimethylthiazole-2-yl]-2,5-diphenyltetrazolium bromide
DMSO: Dimethyl sulfoxide
GICR strain: Cell growth test was performed using E6R (Stem Cells. 2016 Aug; 34(8): 2016-25.).

Cell culture solutions were obtained by mixing a medium prepared by adding various compounds, bFGF (Peprotech, 100-18B) and EGF (Peprotech, 100-15) to DMEM/Ham's F-12 medium (SIGMA, D8062) and a medium prepared by adding FCS (Hyclone, SH30910.03) to DMEM medium (Nacalai tesque, 08458-45) to a concentration of 10% for use.

Each compound adjusted to a predetermined concentration was dispensed into a 96-well plate (Falcon, 353072) at 80 μL/well, and E6 cells and E6R cells were seeded on the 96-well plate at 1000 cells/80 μL/well. After 3 days, 10 μL of an MTT reagent (DOJINDO, 341-01823) was added, the cells were incubated at 37°C for 3 hours, the culture solution was removed, and 100 μL of DMSO was added. The absorbance at 570 nm was measured using Bio-Rad iMark (Bio-Rad, 168-1130JA). The growth inhibition rate at each test compound concentration was calculated by the following formula using the absorbance as an index of the viable cell count.

The growth inhibition rate at each compound concentration was determined, and the 50% growth inhibition concentration GI50 (μmol/L)] was calculated using XLfit.

$$\text{Growth inhibition rate (\%)} = \text{(Amount of luminescence from wells supplemented with test compound)} / \text{(Amount of luminescence from wells supplemented with DMSO)} \times 100$$

The results are shown in Tables 4 to 6.

The abbreviations in the table have the following meanings.

A: GI50 value < 1.0 μmol/L
B: 1.0 μmol/L ≤ GI50 value <10 μmol/L
C: 10 μmol/L ≤ GI50value

[Table 4]

| Co. No. | Structural Formula | ER6 GI50(nmol/L) | Evaluation |
|---|---|---|---|
| 1 | 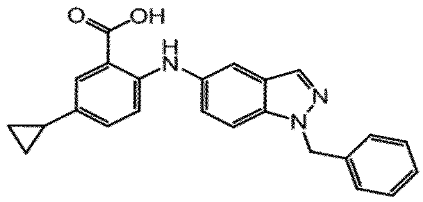 | 30.61 | A |
| 2 | 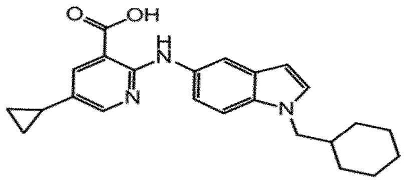 | 82.61 | A |
| 3 | 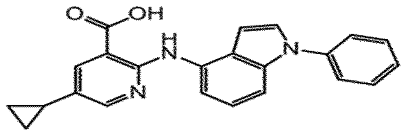 | 11.04 | A |
| 4 | 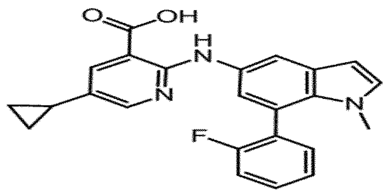 | 1.69 | A |
| 5 | 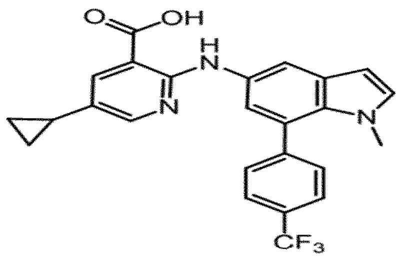 | <0.14 | A |
| 6 | 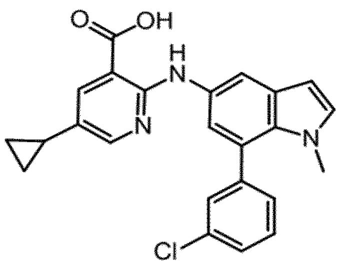 | 2.23 | A |
| 7 | 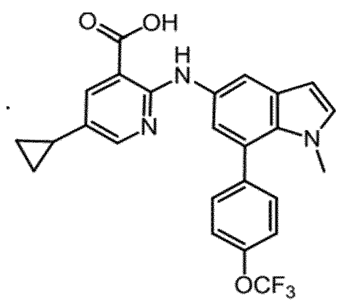 | <0.14 | A |

[Table 5]

| Co. No. | Structural Formula | ER6 GI50(nmol/L) | Evaluation |
|---|---|---|---|
| 8 | 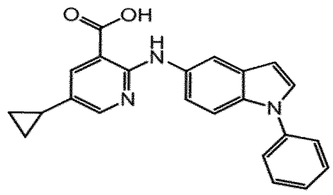 | 11.76 | A |
| 9 | 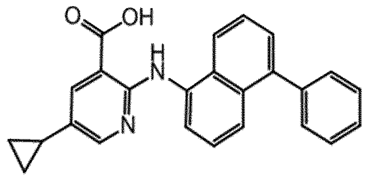 | 4.35 | A |
| 10 | 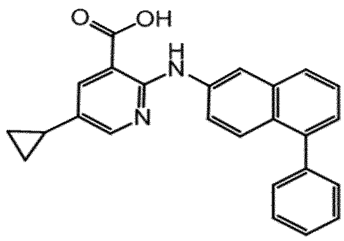 | 1.77 | A |
| 11 | 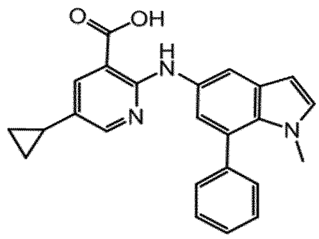 | 4.77 | A |
| 12 | 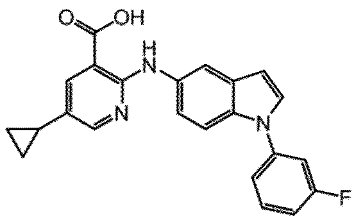 | 23.85 | A |
| 13 | 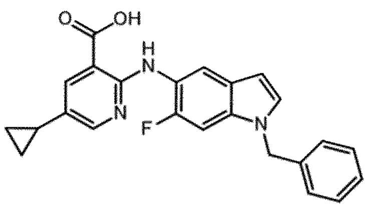 | 11.41 | A |
| 14 | 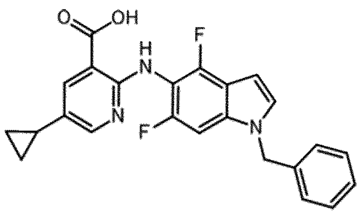 | 47.56 | A |

[Table 6]

| Co. No. | Structural Formula | ER6 GI50(nmol/L) | Evaluation |
|---|---|---|---|
| 15 | 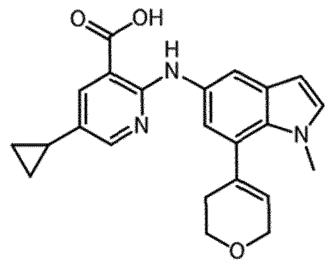 | 25.92 | A |
| 16 | 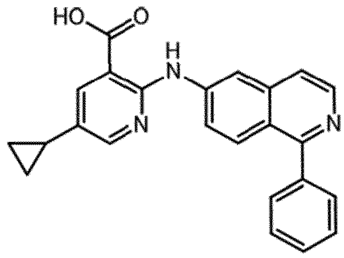 | 3.19 | A |
| 17 | 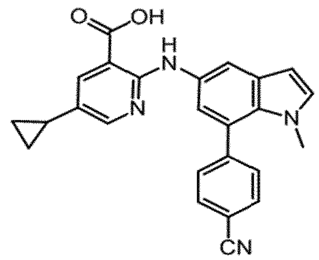 | 2.96 | A |
| 18 | 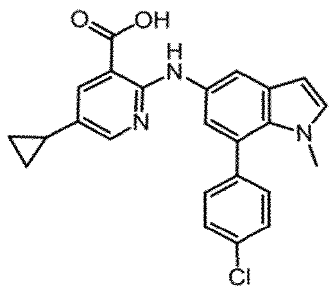 | 0.62 | A |
| 19 | 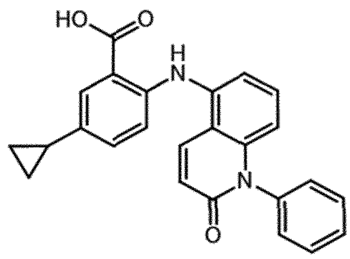 | 5.80 | A |

(continued)

| Co. No. | Structural Formula | ER6 GI50(nmol/L) | Evaluation |
|---|---|---|---|
| 20 | 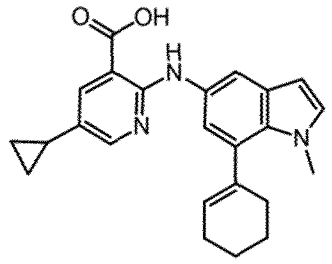 | 0.36 | A |

Compound Nos. 1 to 20 showed an excellent cell growth inhibitory effect.

[Compound 8 promotes SOX2 expression reduction and extranuclear elimination through inhibition of DHODH function, and extranuclear elimination of SOX2 is CRM1-dependent]

The GIC strains (E6 cell: black; E16 cell: white) were treated with Compound 8 (10 μM), and a neural stem cell marker (SOX2, NESTIN), a differentiation marker (βIII tubulin, Glial fibrillary Acidic Protein (GFAP), Galactocerebroside (GC)), and another differentiation marker (B: βIII tubulin (green), Glial fibrillary Acidic Protein (GFAP, red), Galactocerebroside (GC, green), nucleus ((DAPI staining(blue)) were immunostained, thereby quantitatively determining the positive rate. As a result, a remarkable decrease in SOX2 and NESTIN was observed, and it was clarified that the expression of differentiation markers increased (* $P < 0.05$, ** $P < 0.01$, *** $P < 0.001$, statistical processing is t-test) (Fig. 1). As a result of knocking down the DHODH gene, it was confirmed that the SOX2 signal decreased or disappeared in the knockdown vector-transfected cells (GFP positive, green).

The extranuclear elimination of compound 8-dependent SOX2 is dependent on the extranuclear transport factor, Exportin (also known as CRM1). The cells were cultured for one day in the presence of Compound 8 (10 μM) and a CRM1-specific inhibitor leptomycin B (LMB, 150 nM), and GICs were immunostained with the SOX2 antibody. As a result, the SOX2/DAPI positive rate in GICs (E6) was as follows: Control: 95 ± 2%, supplemented with Compound 8: 7 ± 3%, supplemented with Compound 8 + LMB: 87 ± 9%; the SOX2/DAPI positive rate in GICs (E16) was as follows: Control: 72 ± 11%, supplemented with compound 8: 0.5 ± 1%, supplemented with Compound 8 + LMB: 81 ± 10%. The addition of LMB suppressed Compound 8-dependent extranuclear elimination of SOX2.

[Compound 8 exhibits tumor growth inhibitory effect of GIC in xenograft model and promotes SOX2 expression reduction and extranuclear elimination]

The compound 8 exhibited an antitumor effect in a xenograft model. Temozolomide-resistant E6 (E6R, $1 \times 10^6$) was subcutaneously transplanted into NOD/SCID mice (n = 6), and Compound 8 (1.0 mg/kg) was orally administered for 11 consecutive days after the long diameter and short diameter of tumor became 5 to 6 mm. As a result, reduced tumor growth was confirmed (Fig. 2). The size of tumor was calculated as (long diameter) $\times$ (short diameter)$^2$/2. Tumors were excised from Compound 8 treated mice and control mice on Day 12 from the start of oral administration of Compound 8 and fixed with 4% paraformaldehyde, followed by preparation of frozen sections. As a result, tumor cell aggregates disappeared by treatment with Compound 8. In addition, disappearance of SOX2 in the mouse tumor tissue and an extranuclear staining image were observed by treatment with Compound 8.

[Intranuclear retention of SOX2 partially neutralizes the cytotoxic activity of Compound 8]

When UDP-GlcNAc was added after the chemical was added to GIC, reduced SOX2 expression in GICs and the cytotoxic activity of Compound 8 on GICs were neutralized (Figs. 3 and 4). SOX2 K75A in which lysine 75 involved in the binding of CRM1 and SOX2 was substituted with alanine and SOX2 S248A in which the O-GlcNAcylation target serine 248 was substituted with alanine were prepared, thereby establishing GICs that constitutively express these SOX2 mutants and wild-type SOX2 (SOX2 wt). After treatment with Compound 8, the localization of SOX2 was examined by immunostaining using an anti-FLAG antibody, and the viability of GICs expressing these mutants was examined by MTT assay (Fig. 5). In the presence of Compound 8, SOX2 K75A was present in the nucleus and partially neutralized the cytotoxic activity of Compound 8. SOX2 S248A was not retained in the nucleus by treatment with Compound 8, and no inhibitory effect on cytotoxic activity was observed. Forced expression of SOX2 wt showed an intermediate value

between SOX2 K75A and SOX2 S248A. The SOX2/DAPI positive rate was as follows: GIC (E6) : Control: 0%, SOX2 wt: 36 ± 5%, SOX2 K75A: 63 ± 5%, SOX2 S248A: 18 ± 2%; GIC (E16): Control: 0%, SOX2 wt: 22 ± 4%, SOX2 K75A: 58 ± 6%, SOX2 S248A: 15 ± 3%. Error bar: ± SD. Statistical processing is t-test. *P < 0.05, **P < 0.01, ***P < 0.001. GIC was cultured for 2 days with Compound 8 (10 μmol/L) alone or together with UDP-GlcNAc (1 mmol/L), and immunostaining was performed for SOX2 and NESTIN. As a result, the addition of UDP-GlcNAc suppressed Compound 8-dependent extranuclear elimination of SOX2.

[O-GlcNAcylation of SOX2 is involved in intranuclear retention of SOX2]

OSMI-1, which is an O-GlcNAc transferase (OGT)-specific inhibitor, suppresses GIC growth and SOX2 expression. (A) GICs were cultured for 3 days in the presence of various concentrations of OSMI-1, and cell growth was examined using MTT assay (Fig. 6). (B) The results of immunostaining for SOX2 and NESTIN using GICs cultured for 2 days in the presence of 20 μmol/L OSMI-1 and calculating the SOX2 positive rate (SOX2/DAPI) are shown below.

GIC (E6): Control: 71 ± 2%
Supplemented with OSMI-1: 32 ± 4%
GIC (E16): Control: 65 ± 5%
Supplemented with OSMI-1: 30 ± 5%

[Compounds 8 and 21 inhibit DHODH; Compound 21 exhibits GIC growth inhibitory effect]

For DHODH enzyme assay, "Benjamin Bader, Wolfgang Knecht, Markus Fries, and Monika Löffler. Expression, Purification, and Characterization of Histidine-Tagged Rat and Human Flavoenzyme Dihydroorotate Dehydrogenase. Protein Expression and Purification, 1998, 13, 414-422" was referred to.

DHODH activity was evaluated using an enzyme assay system that couples with an assay in a blue-coloring dye 2,6-dichlorophenol indophenol (DCIP, MP BIOMEDICALS, MP150118) quenching assay. Purified recombinant human DHODH (DHODH, 31-395aa, Human, His tag, E.coli, ATGP1615) was purchased from ATGen. The enzyme assay was performed in a 384-well plate using a buffer solution adjusted to pH 8.0 with the addition of 5N potassium hydroxide (FUJIFILM Wako Pure Chemical Corporation, 168-21815) to 100 mM Hepes (DOJINDO LABORATORIES, 342-01375), 400 mM NaCl (FUJIFILM Wako Pure Chemical Corporation, 191-01665), 10% Glycerol (FUJIFILM Wako Pure Chemical Corporation, 075-00616), 0.05% TritonX-100(Sigma-Aldrich, T8787-100ML), 0.2 mM Ubiquinone-10(FUJIFILM Wako Pure Chemical Corporation, 216-00761), 0.1 mM DHO (Sigma-Aldrich, D7128), 0.5% DMSO (FUJIFILM Wako Pure Chemical Corporation, 047-29353), 0.175 μg/mL DHODH, and 0.12 mM DCIP. A predetermined concentration of each test compound was added using BiomekNX (Beckman Coulter), and the enzymatic reaction was started by adding the substrate. Enzyme activity was assessed by measuring the decrease in DCIP absorbance (600 nm) for 50 minutes using an Envision plate-reading spectrophotometer (PerkinElmer Co., Ltd.).

The enzyme reaction inhibition rate at each test compound concentration was determined, and the 50% enzyme reaction inhibition concentration [IC50 (μmol/L)] was calculated using XLfit.

Enzyme reaction inhibition rate (%) = (Amount of luminescence from wells supplemented with test compound) / (Amount of luminescence from wells supplemented with DMSO) × 100

As shown in Table 7, each test compound showed an excellent enzyme reaction inhibitory effect.

[Table 7]

| Compound No. | Structure | DHODH enzyme inhibition $IC_{50}$ (nmol/L) |
|---|---|---|
| Compound 21 | 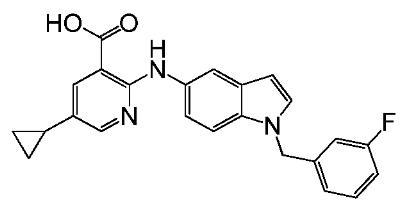 | 28.6 |

(continued)

| Compound No. | Structure | DHODH enzyme inhibition IC $_{50}$ (nmol/L) |
|---|---|---|
| Compound 8 | 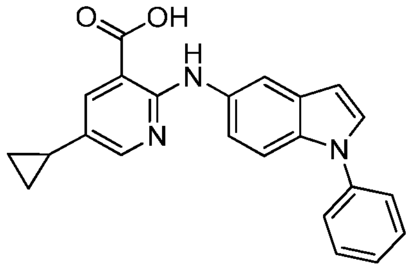 | 8.96 |

[Cell proliferation test]

Abbreviations used herein are shown below.

DMEM: Dulbecco's modified Eagle's medium

bFGF: Basic fibroblast growth factor

EGF: Epidermal growth factor

FCS: Fetal calf serum

MTT: 3-[4,5-dimethylthiazole-2-yl]-2,5-diphenyltetrazolium bromide

DMSO: Dimethyl sulfoxide

GIC and GICR strain: Cell growth test was performed using E6, E6R, E16, and E16R (Stem Cells. 2016 Aug; 34(8): 2016-25.).

Cell culture solutions were obtained by mixing a medium prepared by adding various compounds, bFGF (Peprotech, 100-18B) and EGF (Peprotech, 100-15) to DMEM/Ham's F-12 medium (SIGMA, D8062) and a medium prepared by adding FCS (Hyclone, SH30910.03) to DMEM medium (Nacalai tesque, 08458-45) to a concentration of 10% for use.

Each compound adjusted to a predetermined concentration was dispensed into a 96-well plate (Falcon, 353072) at 80 μL/well, and E6 cells and E6R cells were seeded on the 96-well plate at 1000 cells/80 μL/well. After 3 days, 10 μL of an MTT reagent (DOJINDO, 341-01823) was added, the cells were incubated at 37°C for 3 hours, the culture solution was removed, and 100 μL of DMSO was added. The absorbance at 570 nm was measured using Bio-Rad iMark (Bio-Rad, 168-1130JA). The growth inhibition rate at each test compound concentration was calculated by the following formula using the absorbance as an index of the viable cell count.

The growth inhibition rate at each compound concentration was determined, and the 50% growth inhibition concentration GI50 (μmol/L)] was calculated using XLfit.

$$\text{Growth inhibition rate (\%)} = \text{(Amount of luminescence from wells supplemented with test compound)} / \text{(Amount of luminescence from wells supplemented with DMSO)} \times 100$$

The results are shown in Tables 8 and 9.

The abbreviations in the table have the following meanings.

A: GI50 value < 1.0 μmol/L
B: 1.0 μmol/L ≤ GI50 value < 10 μmol/L
C: 10 μmol/L ≤ GI50 value

[Table 8]

| Compound No. | Structure | $GI_{50}$ (nmol/L) of Glioma-initiating cell E6R |
|---|---|---|
| Compound 21 | 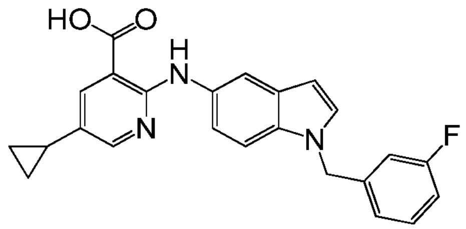 | 654.6 A |

[Table 9]

| Compound No. | Structure | $GI_{50}$ (nmol/L) of Glioma-initiating cell E6 | $GI_{50}$ (nmol/L) of Glioma-initiating cell E16 | $GI_{50}$ (nmol/L) of Glioma-initiating cell E16R |
|---|---|---|---|---|
| Compound 21 | 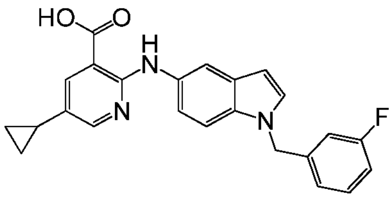 | 100.5 A | 11792.3 C | 1962.4 B |
| Compound 8 | 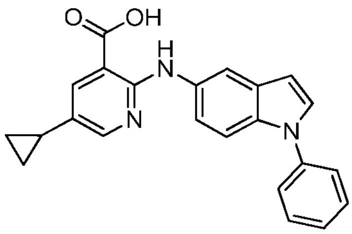 | <7 A | 433.7 A | 489.8 A |

[Compound 8 suppresses the action of DHODH enzyme of GIC and inhibits pyrimidine metabolism]

Compound 8 was added to GICs (E6, E16) at 10 μM and treated for 24 hours. Cells were washed with a 5% mannitol solution, 70% ice-cold methanol was added, and metabolites were extracted. Fig. 7 shows the results of quantitative determination of metabolites using LC-MS/MS. Compound 8 treatment increased the amount of DHO 596-fold in E6 cells and 125-fold in E16 cells as compared to the control DMSO-treated group. In addition, the amount of orotic acid was decreased in E16 cells. Addition of Compound 8 reduced the amounts of pyrimidine metabolites downstream of orotic acid, UMP, UDP, UTP, CMP, CDP, CTP, dCTP, and dTTP while the amounts of purine metabolites, ATP, GTP, dATP, and dGTP remained unchanged. It was revealed that pyrimidine metabolism can be inhibited by inhibiting DHODH.

<Summary>

Compound 8 inhibits DHODH, reduces pyrimidine metabolites, and causes the amount of UDP-GlcNAc to decrease. It was clarified that when the amount of UDP-GlcNAc decreases, the O-GlcNAcylation of SOX2 is reduced, the extranuclear elimination occurs, and the SOX2 expression decreases. Accordingly, GICs are less likely to be undifferentiated and are thus to be differentiated.

Industrial Applicability

Since the compound or a salt thereof of the present invention has an excellent antiproliferative effect, it is useful for preventive or therapeutic treatments of glioma.

**Claims**

1. A glioma treatment agent which comprises a compound represented by formula (1) or a salt thereof:

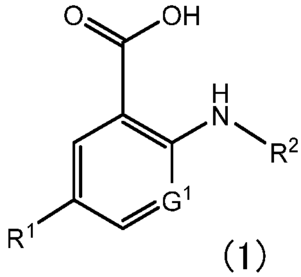

(1)

(wherein

$G^1$ is CH or a nitrogen atom;
$R^1$ is a halogen atom, an optionally substituted $C_{1-6}$ alkyl group, or an optionally substituted $C_{3-8}$ cycloalkyl group;
$R^2$ is an optionally substituted bicyclic condensed hydrocarbon ring group or an optionally substituted bicyclic heterocyclic group, provided that

(1) when $R^2$ is an optionally substituted bicyclic condensed hydrocarbon ring group, $G^1$ is a nitrogen atom;
(2) when $G^1$ is CH, and $R^1$ is a chlorine atom or an optionally substituted $C_{3-8}$ cycloalkyl group, $R^2$ is a group represented by formula (2-1) or (2-2)

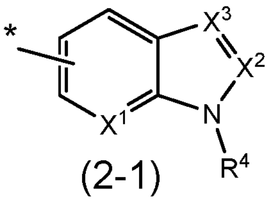

(2-1)

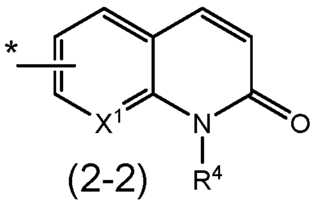

(2-2)

(wherein

$X^1$, $X^2$, and $X^3$ are the same or different and are each $CR^3$ or a nitrogen atom;
$R^3$ is a hydrogen atom, a halogen atom, or an optionally substituted $C_{1-6}$ alkyl group, and $R^4$ is an optionally substituted $C_{3-6}$ alkyl group, an optionally substituted $C_{3-8}$ cycloalkyl group, an optionally substituted $C_{3-8}$ cycloalkyl $C_{1-6}$ alkyl group, an optionally substituted aryl group, an optionally substituted ar-$C_{1-6}$ alkyl group, an optionally substituted acyl group, an optionally substituted heterocyclic group, or an optionally substituted heterocyclic $C_{1-6}$ alkyl group)).

2. The glioma treatment agent comprising the compound or a salt thereof according to claim 1, wherein $R^1$ is a chlorine atom or an optionally substituted $_{3-8}$ cycloalkyl group.

3. The glioma treatment agent comprising the compound or a salt thereof according to claim 1 or 2, wherein $R^2$ is a group represented by formula (3-1) or (3-2)

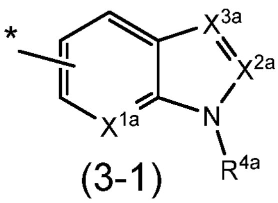

(3-1)

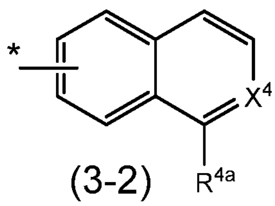

(3-2)

(wherein

$X^{1a}$, $X^{2a}$, and $X^{3a}$ are the same or different and are each $CR^5$ or a nitrogen atom;
$X^4$ is CH or a nitrogen atom;
$R^{4a}$ is an optionally substituted $C_{1-6}$ alkyl group or an optionally substituted aryl group;
$R^5$ is a hydrogen atom, an optionally substituted $C_{3-8}$ cycloalkenyl group, or an optionally substituted aryl group), or

when $G^1$ is CH, and $R^1$ is a chlorine atom or an optionally substituted $C_{3-8}$ cycloalkyl group, $R^2$ is a group represented by formula (4)

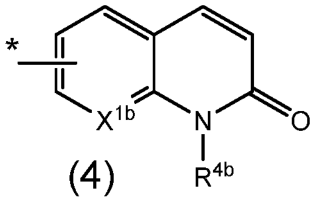

(4)

(wherein

$X^{1b}$ is CH or a nitrogen atom;
$R^{4b}$ is an optionally substituted aryl group).

**4.** The glioma treatment agent comprising the compound or a salt thereof according to any one of claims 1 to 3, wherein $G^1$ is a nitrogen atom, $R^2$ is a group represented by formula (5)

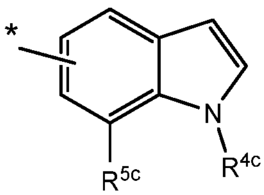

(5)

(wherein

$R^{4c}$ is an optionally substituted $C_{1-6}$ alkyl group;
$R^{5c}$ is an optionally substituted aryl group or an optionally substituted $C_{3-8}$ cycloalkenyl group).

**5.** A pharmaceutical composition containing the treatment agent according to any one of claims 1 to 4.

[Fig. 1]

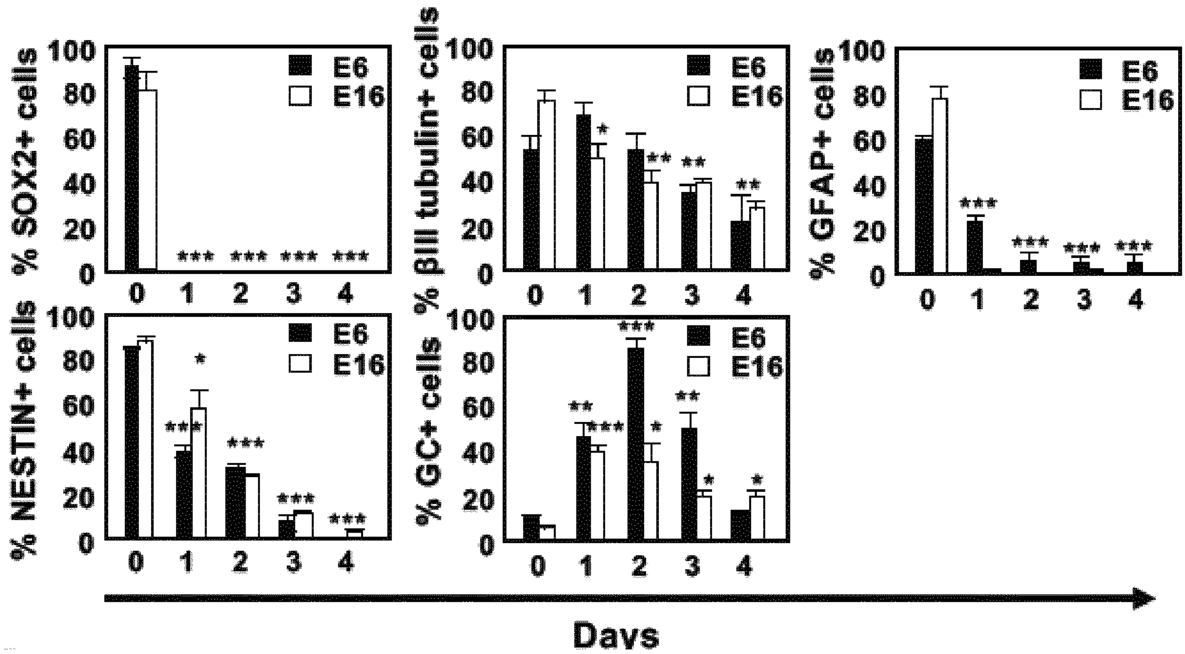
% SOX2+ cells
% βIII tubulin+ cells
% GFAP+ cells
% NESTIN+ cells
% GC+ cells
E6
E16
Days

[Fig. 2]

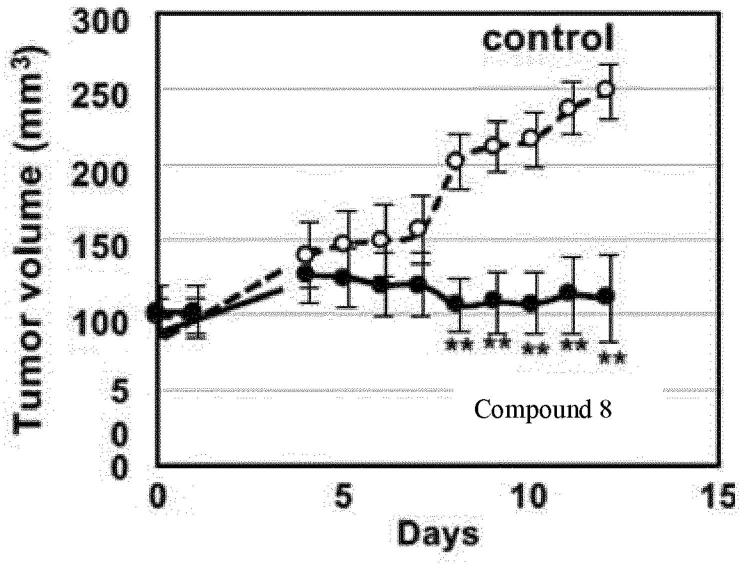
Tumor volume (mm3)
control
Compound 8
Days

[Fig. 3]

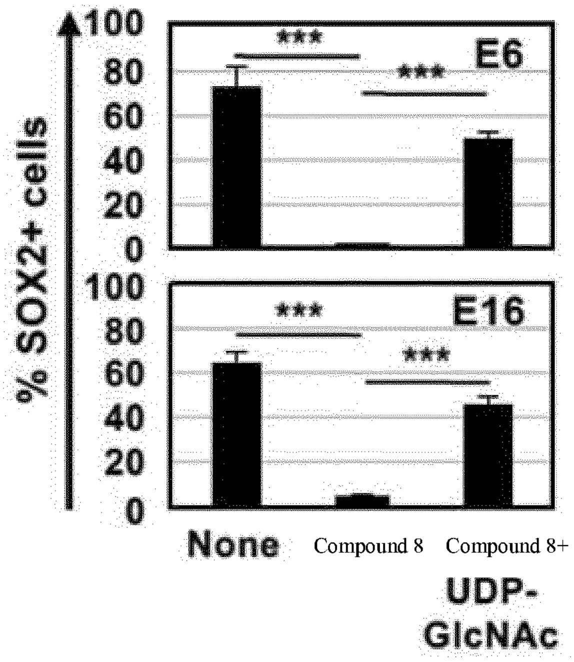


[Fig. 4]

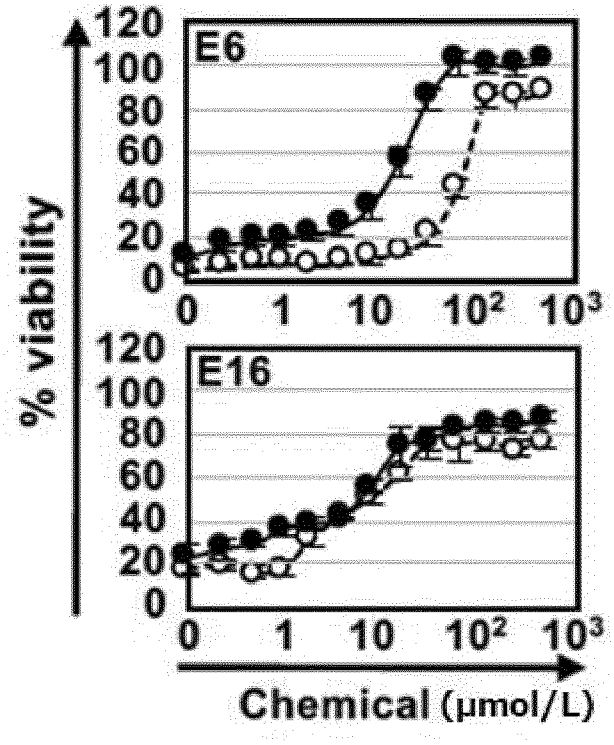


Black circle: UDP-GlcNAc; White circle: UDP

[Fig. 5]

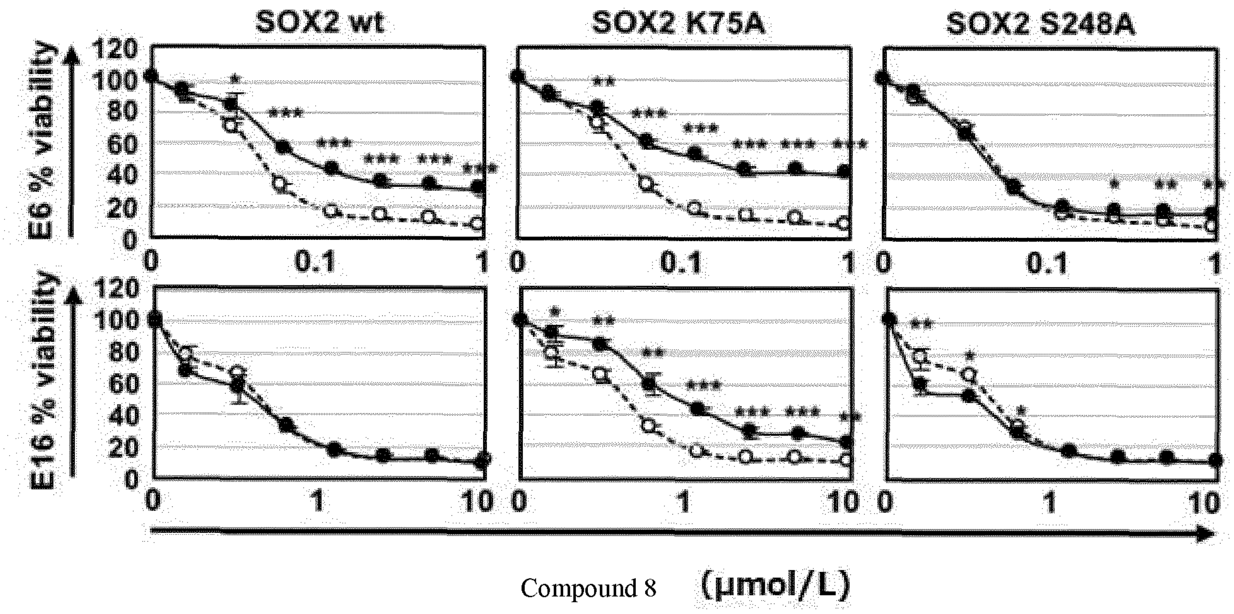


Compound 8

Black circle: Wild type (wt) or mutant-type (K75A, S248A) SOX2 forced expression GIC; White circle: Control GIC

[Fig. 6]

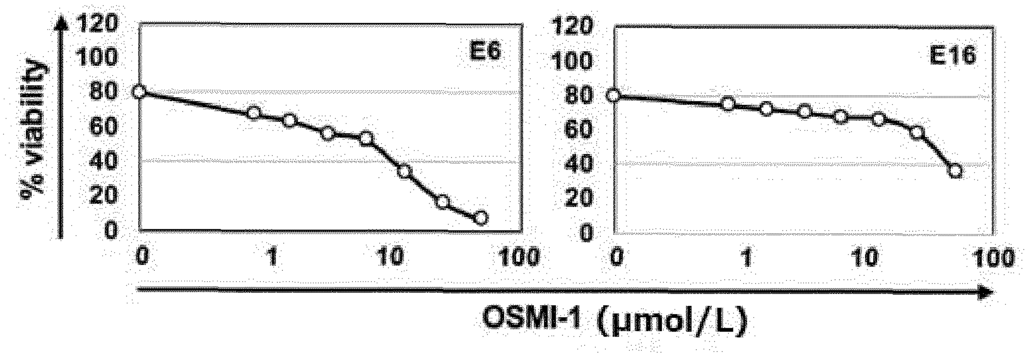

[Fig. 7]

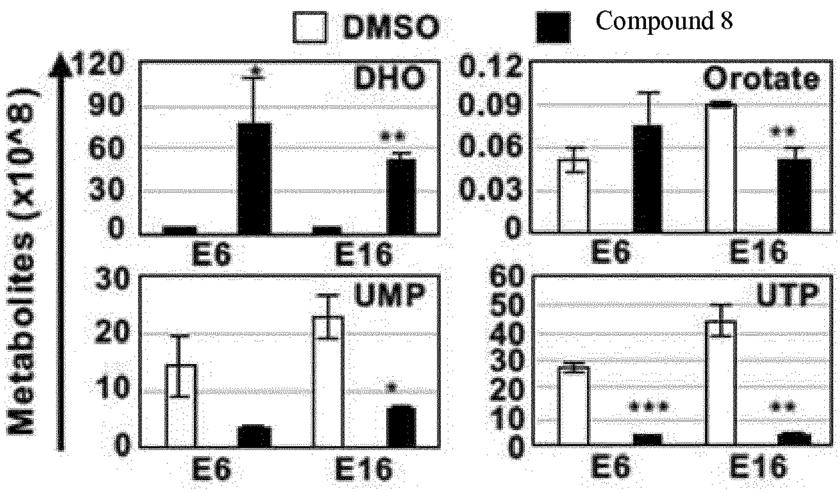
DMSO
Compound 8
Metabolites (x10^8)
DHO
Orotate
UMP
UTP
E6
E16
120
90
60
30
0
0.12
0.09
0.06
0.03
0
30
20
10
0
60
50
40
30
20
10
0
*
**
***

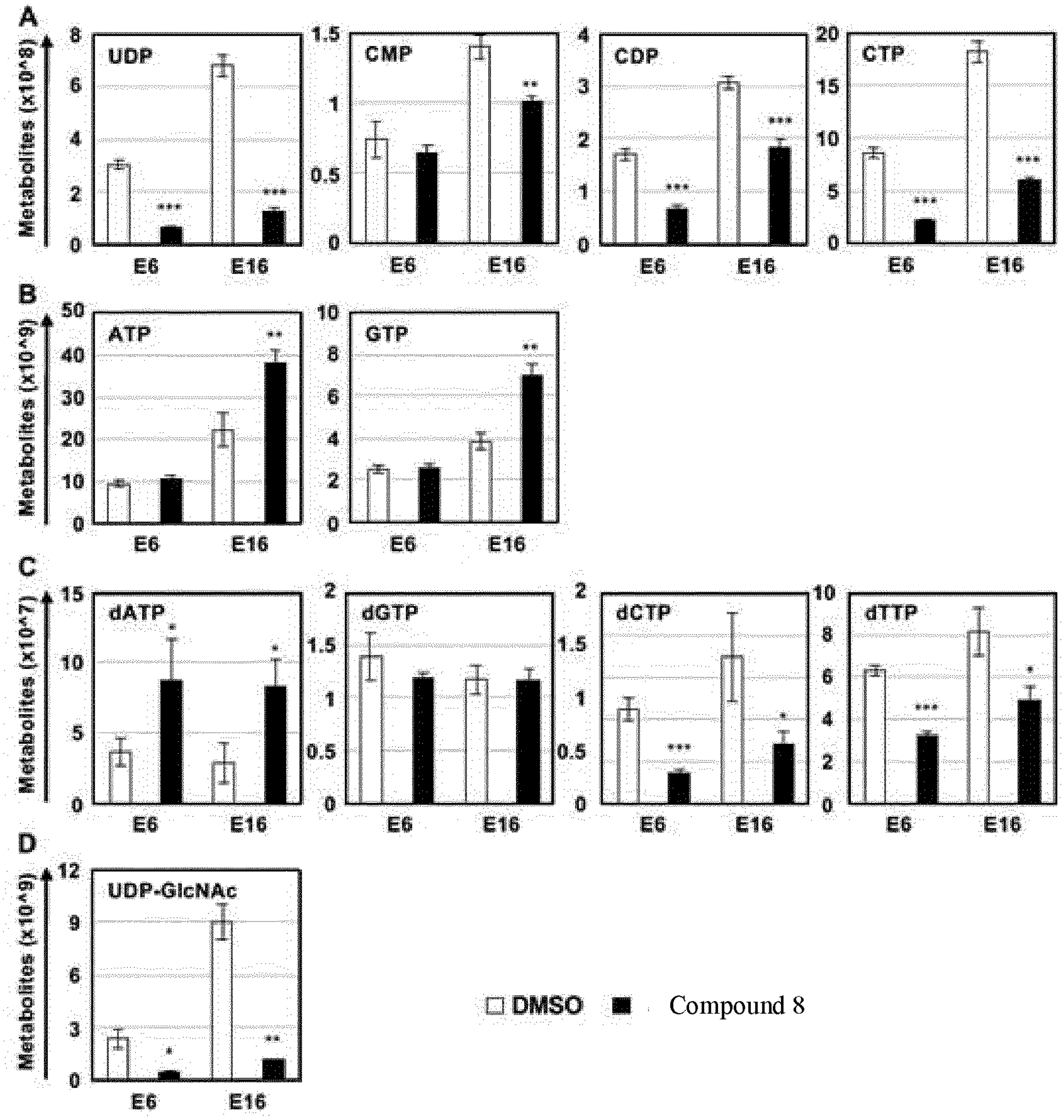
A
Metabolites (x10^8)
UDP
CMP
CDP
CTP
B
Metabolites (x10^9)
ATP
GTP
C
Metabolites (x10^7)
dATP
dGTP
dCTP
dTTP
D
Metabolites (x10^9)
UDP-GlcNAc
E6
E16
DMSO
Compound 8

## INTERNATIONAL SEARCH REPORT

International application No.
PCT/JP2019/022538

A. CLASSIFICATION OF SUBJECT MATTER
Int.Cl. A61K31/4725(2006.01)i, A61K31/416(2006.01)i, A61K31/4418(2006.01)i, A61K31/4439(2006.01)i, A61K31/4704(2006.01)i, A61P35/00(2006.01)i
According to International Patent Classification (IPC) or to both national classification and IPC

B. FIELDS SEARCHED
Minimum documentation searched (classification system followed by classification symbols)
Int.Cl. A61K31/4725, A61K31/416, A61K31/4418, A61K31/4439, A61K31/4704, A61P35/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 2014/069510 A1 (TOYAMA CHEMICAL CO., LTD.) 08 May 2014, claims, paragraph [0041], examples, test examples & US 2015/0299189 A1, claims, paragraphs [0170]-[0173], examples, test examples & EP 2915804 A1 & CA 2890003 A & KR 10-2015-0079916 A & CN 104870422 A & TW 201422605 A | 1-5 |
| X | JP 2010-520268 A (BIOLIPOX AB) 10 June 2010, claims, paragraphs [0036]-[0037], [0141]-[0147], compound numbers 28, 29, 31, example 98 & WO 2008/107661 A1, claims, page 12, line 23 to page 13, line 29, page 45, line 19 to page 47, line 22, compound numbers 28, 29, 31, example 98 & US 2010/0144872 A1 & CA 2680139 A & CN 101646647 A | 1, 3, 5 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 July 2019 (18.07.2019) | 30 July 2019 (30.07.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/022538

C (Continuation). DOCUMENTS CONSIDERED TO BE RELEVANT

| Category* | Citation of document, with indication, where appropriate, of the relevant passages | Relevant to claim No. |
|---|---|---|
| X | JP 2002-540103 A (PARKER HUGHES INSTITUTE) 26 November 2002, claims, paragraph [0005], example 2, compound number 2 (P-88) & WO 2000/056720 A1, claims, page 1, line 33 to page 2, line 8, example 2, compound number 2 (P-88) & US 2001/0016588 A1 & US 2002/0137757 A1 & US 2004/0039002 A1 & US 2005/0075353 A1 & AU 4171900 A & CA 2367861 A | 1, 5 |
| X | WO 2009/021696 A1 (LABORATRIOS ALMIRALL, S.A.) 19 February 2009, claims, page 1, line 1 to page 3, line 21, page 140, line 28 to page 143, line 18, examples 16, 17 & JP 2010-535824 A & US 2011/0212945 A1 & US 2014/0005178 A1 & EP 2100881 A1 & CA 2695475 A & CN 101801931 A & KR 10-2010-0046001 A & TW 200911244 A | 1, 5 |
| X | JP 2001-302667 A (BAYER AG.) 31 October 2001, claims, paragraph [0013], tables 4-18, 4-29, examples 95, 144 & WO 2001/083485 A1, claims, page 12, line 17 to page 13, line 8, tables 4-18, 4-29, examples 95, 144 & US 2004/0054179 A1 & CA 2407531 A & TW 222974 B & CN 1439009 A & KR 10-2003-0009456 A | 1, 5 |
| X | JP 2010-504298 A (F. HOFFMANN-LA ROCHE AG.) 12 February 2010, claims, paragraphs [0010]-[0011], [0124], examples 289, 479 & WO 2008/034736 A2, claims, page 3, line 24 to page 4, line 15, page 30, line 34 to page 31, line 3, examples 289, 479 & US 2008/0081810 A1 & CA 2662838 A & KR 10-2009-0053833 A & CN 101516366 A | 1, 5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014069510 A


### Non-patent literature cited in the description


- *Stem Cells,* August 2016, vol. 34 (8), 2016-25
- **BENJAMIN BADER ; WOLFGANG KNECHT ; MARKUS FRIES ; MONIKA LÖFFLER.** Expression, Purification, and Characterization of Histidine-Tagged Rat and Human Flavoenzyme Dihydroorotate Dehydrogenase. *Protein Expression and Purification,* 1998, vol. 13, 414-422